# EUROPÄISCHE PATENTSCHRIFT

(11) **EP 0 445 581 B1**
(45) Veröffentlichungstag und Bekanntmachung des Hinweises auf die Patenterteilung: **05.05.1993**
(21) Anmeldenummer: 91102431.3
(22) Anmeldetag: 20.02.1991
(51) Int. Cl.: A61K 47/14, C07K 15/00

(54) **Parenteral applizierbares lagerbeständiges immunstimulierendes Präparat, Verfahren zu seiner Herstellung und dessen Verwendung**
Stable immunostimulating preparation for parenteral application, preparation and use thereof
Préparation imunnostimulante stable et susceptible d'être appliquée par voie parenterale, procédé pour sa préparation et utilisation

(30) Priorität: 06.03.1990 DD 338431
(43) Veröffentlichungstag der Anmeldung: 11.09.1991
(73) Patentinhaber: BERLIN-CHEMIE AG, 12489 Berlin (DE)
(72) Erfinder: Wolf, Ingrid, Dr. rer. nat., O-1136 Berlin (DE); Klehr, Gabriele, O-1110 Berlin (DE)
(74) Vertreter: Hansen, Bernd, Dr. Dipl.-Chem.

(56) Entgegenhaltungen:
- EP-A- 0 080 194
- EP-A- 0 307 553
- EP-A- 0 342 962
- EP-A- 0 371 195
- EP-A- 0 410 372
- DD-A- 263 994
- DD-A- 271 267

## Beschreibung

Die Erfindung betrifft ein zur parenteralen Applikation geeignetes lagerungsbeständiges Präparat zur Immunstimulierung in der Humanmedizin und ein Verfahren zu seiner Herstellung. Es findet Anwendung in der pharmazeutischen Industrie.

Indikationsgebiete des Präparates sind
- Immundefizit bei HIV-/AIDS-Patienten;
- Wiederherstellung der Funktionsfähigkeit des Immunsystems bei Krebspatienten nach Chemo- bzw. Strahlentherapie;
- Immunparalyse bei Organtransplantation;
- Wiederherstellung der Funktionsfähigkeit des Immunsystems nach Knochenmarktransplantation;
- Rheumatoidarthritis;
- Psoriasis mit starker Gelenkbeteiligung.

Peptide sind insbesondere in wäßrigen Lösungen in der Regel instabil. Die Hydrolyse der Peptidbindungen führt zur Spaltung des Moleküls in unwirksame Peptidbruchstücke. Höhere Temperaturen führen insbesondere in Gegenwart von Wasser zur Denaturierung und damit zum Auftreten von Ausfällungen, die eine parenterale Applikation unmöglich machen. In Abhängigkeit von der Aminosäuresequenz des Peptides sind jeweils umfangreiche Versuche notwendig, um eine individuelle optimale Rezeptur zu entwickeln, die eine ausreichende Stabilität des Präparates im Verwendbarkeitszeitraum gewährleistet. Splenopentin und Analoga sind ebenso wie andere Peptide instabil. Entsprechend Monographie und pharmazeutischen Gutachten ist für Diacetylsplenopentinhydrochlorid eine sehr kühle, vor Licht und Feuchtigkeit geschützte Aufbewahrung bereits für die Substanz vorgeschrieben. Erfahrungsgemäß verschlechtert sich die Stabilität in Gegenwart von Wasser oder bei der Lyophilisierung.

Lagerungsstabile parenteral applizierte Präparate mit immunstimulierender Wirkung auf der Basis von Splenopentin bzw. seiner Analoga sind bisher nicht bekannt.

Ziel der Erfindung ist die Herstellung eines stabilen parenteral applizierbaren Präparates mit immunstimulierender Wirkung auf der Basis von Splenopentin bzw. Splenopentinanaloga.

Der Erfindung liegt die Aufgabe zugrunde, ein Präparat zur Verfügung zu stellen, das Splenopentin bzw. Splenopentinanaloga als Immunstimulator in einer parenteral applizierbaren lagerungsbeständige Form enthält.

Erfindungsgemäß wird die Aufgabe dadurch gelöst, daß unter Einsatz von Splenopentin bzw. Splenopentinanaloga unter Vermeidung des Fällungsbereiches ein pH-Bereich zwischen 6,0̸ und 8,0̸, vorzugsweise von 6,5 bis 7,5, eingestellt wird, als Peptid vorzugsweise Diacetylsplenopentinhydrochlorid in einer Konzentration von 1 bis 10̸0̸ mg/ml eingesetzt wird und als Stabilitätspromotoren auf 90̸ °C bis 95 °C temperierte wäßrige Lösungen von Hydroxybenzoesäureestern in einem Mischungsverhältnis 1 : 1 bis 10̸ : 1, vorzugsweise 5 : 1, eingesetzt werden. Das Peptid wird in 0̸,1 Mol/l Natronlauge gelöst und anschließend mit der auf 90̸ °C bis 95 °C temperierten Hydroxybenzoesäurelösung versetzt. Nach der pH-Wert-Einstellung wird die Lösung über 0̸,2-µm-Membranfilter steril filtriert und unter aseptischen Bedingungen in 2-ml-Ampullen abgefüllt. Der Hydroxybenzoesäureester wird vorzugsweise in einer Konzentration von 0̸,1 bis 10̸ mg/ml eingesetzt.
Weitere Ausführungsformen der Erfindung sind in den abhängigen Patentansprüchen angegeben.
Die nach dieser Verfahrensweise hergestellten Peptidpräparate zeigen eine gute Stabilität und ermöglichen so auch eine parenterale Anwendung.
Der Einsatz von Hydroxybenzoesäureestern als Lösungsvermittler und -stabilisator für Peptide ist neu, der Erfolg durchaus überraschend.

Die erfindungsgemäße Lösung ermöglicht es, die Stabilität einer wäßrigen Lösung und eines entsprechenden Lyophilisates so zu verbessern, daß keine Einschränkungen der Aufbewahrungsvorschriften mehr erforderlich sind und die resultierenden Lösungen sogar parenteral applizierbar sind.
Die für den Wirkstoff vorgeschriebene Einschränkung der Aufbewahrungsbedingungen (sehr kühle Lagerung und Schutz vor Feuchtigkeit) entfällt.

### Ausführungsbeispiele

### Beispiel 1

In 60̸0̸ ml 0̸,0̸2 Mol/l Phosphorsäure werden 27 g Diacetylsplenopentinhydrochlorid gelöst. Durch einen Überschuß an Natriumhydroxid wird zunächst ein pH-Wert von 8,0̸ bis 9,0̸ eingestellt. Nach 20̸ min Rühren wird mit 0̸,0̸2 Mol/l Phosphorsäure auf pH 7,5 eingestellt. In 30̸0̸ ml Wasser zur Injektion werden 1,5 g Hydroxybenzoesäuremethylester bei 90̸ °C bis 95 °C gelöst und sofort und unter Rühren mit dem Ansatz vereinigt. Es erfolgt eine Feineinstellung des pH-Wertes auf 6,9 - 7,1. Anschließend wird der Ansatz auf 1 l aufgefüllt, über 0̸,2-µm-Membranfilter sterilfiltriert und unter aseptischen Bedingungen konfektioniert.

### Beispiel 2

In 50̸0̸ ml Wasser zur Injektion werden 27 g Splenopentinhydrochlorid gelöst. Der pH-Wert der Lösung wird zunächst mit Meglumin auf 8,0̸ bis 9,0̸ und nach 20̸ min Rühren mit 1 Mol/l Salzsäure auf pH 7,5 eingestellt. In 20̸0̸ ml Wasser zur Injektion werden 0̸,75 g Hydroxybenzoesäurepropylester bei 90̸ °C bis 95 °C gelöst und sofort unter Rühren mit der Peptidlösung vermischt. Nach der Zugabe von 20̸0̸ g Propylenglycol wird der Ansatz auf 1 l aufgefüllt, über 0̸,2-µm-Membranfilter sterilisiert und unter aseptischen Bedingungen abgefüllt.

### Beispiel 3

20̸ g Diacetylsplenopentinhydrochlorid werden in 450̸ ml 0̸,0̸5 Mol/l Natronlauge gelöst. Anschließend werden 50̸ g Alanin eingetragen.
In 450̸ ml Wasser zur Injektion werden 1,5 g Hydroxybenzoesäuremethylester bei 90̸ °C bis 95 °C gelöst und sofort mit der Peptidlösung vermischt. Nach 20̸minütigem Rühren wird der pH-Wert mit Salzsäure auf pH 7,5 eingestellt und das Volumen mit Wasser zur Injektion auf 1 l ergänzt.
Die über Membranfilter sterilfiltrierte Injektionslösung wird unter aseptischen Bedingungen in Injektionsflaschen abgefüllt und bei Temperaturen bis 25 °C lyophilisiert.

## Patentansprüche (Patentansprüche für folgende(n) Vertragsstaat(en): AT, BE, CH, DE, FR, GB, IT, LI, NL, SE)

1. Parenteral applizierbares lagerbeständiges immunstimulierendes Präparat, dadurch gekennzeichnet, daß es aus einer auf einen pH-Wert zwischen 6,0̸ und 8,0̸ gepufferten wäßrigen oder lösungsmittelhaltigen Splenopentin- bzw. Splenopentinanalogonlösung mit einem Zusatz von Hydroxybenzoesäureester besteht.

2. Präparat nach Anspruch 1, dadurch gekennzeichnet, daß es als Peptid Diacetylsplenopentinhydrochlorid in einer Konzentration von 1 bis 10̸0̸ mg/ml und Hydroxybenzoesäureester in Konzentrationen von 0̸,1 bis 10̸ mg/ml enthält.

3. Präparat nach Anspruch 1 oder 2, dadurch gekennzeichnet, daß die Peptidlösung mit üblichen pharmazeutisch und pharmakologisch geeigneten Puffersubstanzen auf einen pH-Wert von 6,5 bis 7,5 gepuffert ist.

4. Präparat nach einem der Ansprüche 1 bis 3, dadurch gekennzeichnet, daß das Mischungsverhältnis von Peptidlösung und zugesetzter Hydroxybenzoesäurelösung 1 : 10̸ bis 10̸ : 1, vorzugsweise 5 : 1, beträgt.

5. Präparat nach einem der Ansprüche 1 bis 4, dadurch gekennzeichnet, daß die Lösung als organisches Lösungsmittel Propylenglycol und/oder Ethanol enthält.

6. Präparat nach einem der Ansprüche 1 bis 5, dadurch gekennzeichnet, daß die Lösung 1 % bis 10̸ % eines Lyophilisierungsvehikels, bevorzugt Mannitol oder Alanin, enthält und bei Temperaturen bis 30̸°C lyophilisiert ist.

7. Verfahren zur Herstellung eines parenteral applizierbaren lagerbeständigen immunstimulierenden Präparates, dadurch gekennzeichnet, daß eine auf einen pH-Wert zwischen 6,0̸ und 8,0̸ gepufferte wäßrige oder lösungsmittelhaltige Splenopentin- bzw. Splenopentinanalogonlösung mit einer auf 90̸ °C bis 95 °C erwärmten wäßrigen Hydroxybenzoesäureesterlösung versetzt und nach üblicher pharmazeutischer Technologie sterilisiert und abgefüllt bzw. lyophilisiert wird.

8. Verfahren nach Anspruch 7, dadurch gekennzeichnet, daß als Peptid Diacetylsplenopentinhydrochlorid in einer Konzentration von 1 bis 10̸0̸ mg/ml verwendet wird und der Hydroxybenzoesäureester in Konzentrationen von 0̸,1 bis 10̸ mg/ml eingesetzt wird.

9. Verfahren nach Anspruch 7 oder 8, dadurch gekennzeichnet, daß die Peptidlösung mit üblichen pharmazeutisch und pharmakologisch geeigneten Puffersubstanzen auf einen pH-Wert von 6,5 bis 7,5 gepuffert wird.

10. Verfahren nach einem der Ansprüche 7 bis 9, dadurch gekennzeichnet, daß das Mischungsverhältnis von Peptidlösung und Hydroxybenzoesäurelösung 1 : 1 bis 10̸ : 1, vorzugsweise 5 : 1, ist.

11. Verfahren nach einem der Ansprüche 7 bis 10, dadurch gekennzeichnet, daß der Lösung als organisches Lösungsmittel Propylenglycol und/oder Ethanol hinzugefügt wird.

12. Verfahren nach einem der Ansprüche 7 bis 11, dadurch gekennzeichnet, daß der Lösung 1 % bis 10̸ % eines Lyophilisationsvehikels, bevorzugt Mannitol oder Alanin, hinzugefügt und anschließend bei Temperaturen bis 30̸ °C lyophilisiert wird.

13. Verwendung eines lagerbeständigen Präparates nach einem der Ansprüche 1 bis 6 zur Herstellung eines Medikamentes zur Immunstabilisierung.

## Patentansprüche (Patentansprüche für folgende(n) Vertragsstaat(en): ES)

1. Verfahren zur Herstellung eines parenteral applizierbaren lagerbeständigen immunstimulierenden Präparates, dadurch gekennzeichnet, daß eine auf einen pH-Wert zwischen 6,0̸ und 8,0̸ gepufferte wäßrige oder lösungsmittelhaltige Splenopentin- bzw. Splenopentinanalogonlösung mit einer auf 90̸ °C bis 95 °C erwärmten wäßrigen Hydroxybensoesäureesterlösung versetzt und nach üblicher pharmazeutischer Technologie sterilisiert und abgefüllt bzw. lyophilisiert wird.

2. Verfahren nach Anspruch 1, dadurch gekennzeichnet, daß als Peptid Diacetylsplenopentinhydrochlorid in einer Konzentration von 1 bis 10̸0̸ mg/ml verwendet wird und der Hydroxybenzoesäureester in Konzentrationen von 0̸,1 bis 10̸ mg/ml eingesetzt wird.

3. Verfahren nach Anspruch 1 oder 2, dadurch gekennzeichnet, daß die Peptidlösung mit üblichen pharmazeutisch und pharmakologisch geeigneten Puffersubstanzen auf einen pH-Wert von 6,5 bis 7,5 gepuffert wird.

4. Verfahren nach einem der Ansprüche 1 bis 3, dadurch gekennzeichnet, daß das Mischungsverhältnis von Peptidlösung und Hydroxybenzoesäurelösung 1 : 1 bis 10̸ : 1, vorzugsweise 5 : 1, ist.

5. Verfahren nach einem der Ansprüche 1 bis 4, dadurch gekennzeichnet, daß der Lösung als organisches Lösungsmittel Propylenglycol und/oder Ethanol hinzugefügt wird.

6. Verfahren nach einem der Ansprüche 1 bis 5, dadurch gekennzeichnet, daß der Lösung 1 % bis 10̸ % eines Lyophilisationsvehikels, bevorzugt Mannitol oder Alanin, hinzugefügt und anschließend bei Temperaturen bis 30̸ °C lyophilisiert wird.

## Claims (Claims for the following Contracting State(s): AT, BE, CH, DE, FR, GB, IT, LI, NL, SE)

1. Storage-stable immunostimulating preparation which can be administered parenterally, characterised in that it consists of an aqueous or solvent-containing splenopentin solution or splenopentin analogue solution buffered to a pH value between 6.0 and 8.0 with added hydroxybenzoate.

2. Preparation according to claim 1, characterised in that it contains diacetylsplenopentin hydrochloride as the peptide at a concentration of 1 to 100 mg/ml and hydroxybenzoate at concentrations of 0.1 to 10 mg/ml.

3. Preparation according to claim 1 or 2, characterised in that the peptide solution is buffered to a pH value of 6.5 to 7.5 using conventional pharmaceutically and pharmacologically suitable buffer substances.

4. Preparation according to one of claims 1 to 3, characterised in that the mixing ratio of peptide solution and added hydroxy benzoic acid solution is 1 : 10 to 10 : 1, preferably 5 : 1.

5. Preparation according to one of claims 1 to 4, characterised in that the solution contains propylene glycol and/or ethanol as organic solvent.

6. Preparation according to one of claims 1 to 5, characterised in that the solution contains 1 % to 10 % of a lyophilisation vehicle, preferably mannitol or alanine, and is lyophilised at temperatures up to 30°C.

7. Process for producing a storage-stable immunostimulating preparation which can be administered parenterally, characterised in that an aqueous or solvent-containing splenopentin solution or splenopentin analogue solution buffered to a pH value between 6.0 and 8.0 is treated with an aqueous hydroxybenzoate solution heated at 90°C to 95°C and the mixture is sterilised and filled into ampoules or lyophilised using conventional pharmaceutical technology.

8. Process according to claim 7, characterised in that diacetylsplenopentin hydrochloride is used as the peptide at a concentration of 1 to 100 mg/ml and the hydroxybenzoate is used at concentrations of 0.1 to 10 mg/ml.

9. Process according to claim 7 or 8, characterised in that the peptide solution is buffered to a pH value of 6.5 to 7.5 using conventional pharmaceutically and pharmacologically suitable buffer substances.

10. Process according to one of claims 7 to 9, characterised in that the mixing ratio of peptide solution and hydroxy benzoic acid solution is 1 : 1 to 10 : 1, preferably 5 : 1.

11. Process according to one of claims 7 to 10, characterised in that propylene glycol and/or ethanol is added to the solution as organic solvent.

12. Process according to one of claims 7 to 11, characterised in that 1 % to 10 % of a lyophilisation vehicle, preferably mannitol or alanine, is added to the solution and the mixture is then lyophilised at temperatures up to 30°C.

13. Use of a storage-stable preparation according to one of claims 1 to 6 for producing a medicine for immunostabilisation.

## Claims (Claims for the following Contracting State(s): ES)

1. Process for producing a storage-stable immunostimulating preparation which can be administered parenterally, characterised in that an aqueous or solvent-containing splenopentin solution or splenopentin analogue solution buffered to a pH value between 6.0 and 8.0 is treated with an aqueous hydroxybenzoate solution heated at 90°C to 95°C and the mixture is sterilised and filled into ampoules or lyophilised using conventional pharmaceutical technology.

2. Process according to claim 1, characterised in that diacetylsplenopentin hydrochloride is used as the peptide at a concentration of 1 to 100 mg/ml and the hydroxybenzoate is used at concentrations of 0.1 to 10 mg/ml.

3. Process according to claim 1 or 2, characterised in that the peptide solution is buffered to a pH value of 6.5 to 7.5 using conventional pharmaceutically and pharmacologically suitable buffer substances.

4. Process according to one of claims 1 to 3, characterised in that the mixing ratio of peptide solution and hydroxy benzoic acid solution is 1 : 1 to 10 : 1, preferably 5 : 1.

5. Process according to one of claims 1 to 4, characterised in that propylene glycol and/or ethanol is added to the solution as organic solvent.

6. Process according to one of claims 1 to 5, characterised in that 1 % to 10 % of a lyophilisation vehicle, preferably mannitol or alanine, is added to the solution and the mixture is then lyophilised at temperatures up to 30°C.

## Revendications (Revendications pour l'(les) Etat(s) contractant(s) suivant(s): AT, BE, CH, DE, FR, GB, IT, LI, NL, SE)

1. Préparation immunostimulante applicable par voie parentérale, stable au stockage, caractérisée en ce qu'elle se compose d'une solution de splénopentine ou d'analogue de la splénopentine, aqueuse ou contenant un solvant, tamponnée à un pH compris entre 6,0 et 8,0, avec une addition d'ester de l'acide hydroxybenzoïque.

2. Préparation selon la revendication 1, caractérisée en ce qu'elle contient comme peptide du chlorhydrate de diacétylsplénopentine à une concentration de 1 à 100 mg/ml et un ester hydroxybenzoïque à des concentrations de 0,1 à 10 mg/ml.

3. Préparation selon les revendications 1 ou 2, caractérisée en ce que la solution de peptide est tamponnée à un pH de 6,5 à 7,5 avec des substances tampons usuelles appropriées à l'utilisation pharmaceutique et pharmacologique.

4. Préparation selon l'une quelconque des revendications 1 à 3, caractérisée en ce que le rapport de mélange de la solution de peptide et de la solution d'acide hydroxybenzoïque ajoutée est de 1 : 10 à 10 : 1, de préférence de 5 : 1.

5. Préparation selon l'une quelconque des revendications 1 à 4, caractérisée en ce que la solution contient, comme solvant organique, du propylèneglycol et/ou de l'éthanol.

6. Préparation selon l'une quelconque des revendications 1 à 5, caractérisée en ce la solution contient 1 % à 10 % d'un véhicule de lyophilisation, de préférence le mannitol ou l'alanine, et est lyophilisée à des températures allant jusqu'à 30°C.

7. Procédé pour la fabrication d'une préparation immunostimulante applicable par voie parentérale, stable au stockage, caractérisé en ce qu'une solution de splénopentine ou d'analogue de la splénopentine, aqueuse ou contenant un solvant, tamponnée à un pH de 6,0 à 8,0, est additionnée d'une solution aqueuse d'ester hydroxybenzoïque chauffée à 90°C à 95°C, et en ce qu'elle est stérilisée selon la technologie pharmaceutique habituelle et transvasée ou lyophilisée.

8. Procédé selon la revendication 7, caractérisé en ce qu'on utilise comme peptide du chlorhydrate de diacéthylsplénopentine à une concentration de 1 à 100 mg/ml et en ce qu'on utilise l'ester hydroxybenzoïque à des concentrations de 0,1 à 10 mg/ml.

9. Procédé selon les revendications 7 ou 8, caractérisé en ce que la solution de peptide est tamponnée à un pH de 6,5 à 7,5 avec des substances tampons usuelles appropriées à l'utilisation pharmaceutique et pharmacologique.

10. Procédé selon l'une quelconque des revendications 7 à 9, caractérisé en ce que le rapport de mélange de la solution de peptide et de la solution d'acide hydroxybenzoïque est de 1 : 1 à 10 : 1, de préférence de 5 : 1.

11. Procédé selon l'une quelconque des revendications 7 à 10, caractérisé en ce qu'on ajoute comme solvant organique à la solution du propylèneglycol et/ou de l'éthanol.

12. Procédé selon l'une quelconque des revendications 7 à 11, caractérisé en ce qu'on ajoute à la solution 1 % à 10 % d'un véhicule de lyophilisation, de préférence le mannitol ou l'alanine, puis en ce qu'on la lyophilise à des températures allant jusqu'à 30°C.

13. Utilisation d'une préparation stable au stockage selon l'une quelconque des revendications 1 à 6, pour la préparation d'un médicament pour l'immunostabilisation.

## Revendications (Revendications pour l'(les) Etat(s) contractant(s) suivant(s): ES)

1. Procédé de fabrication d'une préparation immunostimulante applicable par voie parentérale, stable au stockage, caractérisé en ce qu'on additionne une solution de splénopentine ou d'analogue de la splénopentine, tamponnée à un pH compris entre 6,0 et 8,0, d'une solution aqueuse d'ester hydroxybenzoïque chauffée à 90°C-95°C, en ce qu'on la stérilise conformément à la technologie pharmaceutique habituelle et en ce qu'on la transvase ou en ce qu'on la lyophilise.

2. Procédé selon la revendication 1, caractérisé en ce qu'on utilise comme peptide le chlorhydrate de diacétylsplénopentine à une concentration de 1 à 100 mg/ml et l'ester hydroxybenzoïque à une concentration de 0,1 à 10 mg/ml.

3. Procédé selon les revendications 1 ou 2, caractérisé en ce que la solution de peptide est tamponnée à un pH de 6,5 à 7,5 au moyen de substances tampons appropriées à l'utilisation pharmaceutique et pharmacologique.

4. Procédé selon l'une quelconque des revendications 1 à 3, caractérisé en ce que le rapport de mélange de la solution de peptide et de la solution d'acide hydroxybenzoïque est de 1 : 1 à 10 : 1, de préférence de 5 : 1.

5. Procédé selon l'une quelconque des revendications 1 à 4, caractérisé en ce qu'on ajoute à la solution, comme solvant organique, du propylèneglycol et/ou de l'éthanol.

6. Procédé selon l'une quelconque des revendications 1 à 5, caractérisé en ce qu'on ajoute à la solution 1 % à 10 % d'un véhicule de lyophilisation, de préférence le mannitol ou l'alanine, puis on la lyophilise à des températures allant jusqu'à 30°C.
